# EUROPÄISCHE PATENTSCHRIFT

(11) **EP 3 628 726 B1**
(45) Veröffentlichungstag und Bekanntmachung des Hinweises auf die Patenterteilung: **16.07.2025**
(21) Anmeldenummer: 18197350.4
(22) Anmeldetag: 27.09.2018
(51) Int. Cl.: C11D 17/00, E03D 9/03, A61L 9/05, A61L 9/12, C11D 17/04, E03D 9/02

(54) **WC-STEIN UMFASSEND ZWEI UNTERSCHIEDLICHE ZUSAMMENSETZUNGEN**
TOILET BOWL DEODORIZER BLOCK COMPRISING TWO DIFFERENT COMPOSITIONS
BLOC DÉSODORISANT POUR WC COMPRENANT DEUX COMPOSITIONS DIFFÉRENTES

(43) Veröffentlichungstag der Anmeldung: 01.04.2020
(73) Patentinhaber: Henkel AG & Co. KGaA, 40589 Düsseldorf (DE)
(72) Erfinder: Holderbaum, Thomas, 40723 Hilden (DE); Segler, Tobias, 40589 Düsseldorf (DE); Schymitzek, Tatiana, 47799 Krefeld (DE)

(56) Entgegenhaltungen:
- WO-A1-00/23558
- DE-A1- 102015 215 135
- GB-A- 2 333 778

## Beschreibung

WC-Reinigungsstücke, auch als WC-Steine bekannt, werden bereits seit langem zur Reinigung, Desinfektion und Beduftung von Toiletten unter dem Beckenrand (sogenannte Rimblocks) sowie im Wasserkasten (In-tank-Blocks oder cistern blocks) eingesetzt. Dabei haben in den letzten Jahren die Ästhetik und die Leistung eine immer größere Bedeutung erlangt. Dies führte beispielsweise zur Entwicklung fester oder gelförmiger oder flüssiger Duftspüler, die teilweise in Mehrkammerbehältnissen angeboten werden und so die Kombination eines Reinigungsmittels, welches bei Betätigung der WC-Spülung abgegeben wird, mit einer permanenten Raumbeduftung erlauben.

Üblicherweise wird ein Rimblock aus einer einzigen Zusammensetzung gebildet. Dabei wird die Zusammensetzung extrudiert, der Strang geschnitten und gegebenenfalls geformt.

Seit einiger Zeit sind auch Rimblocks bekannt, die zwei unterschiedliche Zusammensetzungen aufweisen. Hierdurch wird ermöglicht, unterschiedliche Aktivstoffe, wie Farbstoffe, Duftstoffe in Form von Parfümölen, oder Biozide in dem WC-Block zu kombinieren. Durch einen WC-Block mit mehreren Zusammensetzungen kann ein anderes Freisetzungsprofil erzeugt werden, als dies im Falle eines WC-Blocks mit einer Zusammensetzung möglich ist. Durch Verwendung von wenigstens zwei Zusammensetzungen kann in der ersten Zusammensetzung ein erster Aktivstoff verwendet werden und in der zweiten Zusammensetzung ein zweiter Aktivstoff. Wenn z.B. nur der erste Aktivstoff mit der ersten Zusammensetzung kompatibel ist, d.h. aus chemischen Gründen oder physikochemischen Gründen, der zweite Aktivstoff nicht in der ersten Zusammensetzung eingesetzt werden kann, können durch den Einsatz einer zusätzlichen zweiten Zusammensetzung, die den zweiten Aktivstoff enthält, dennoch beide Aktivstoffe in dem WC-Stein eingesetzt werden.

Aus dem Stand der Technik sind Rimblocks bekannt, die zwei unterschiedliche Zusammensetzungen aufweisen. Allerdings werden diese Steine durch konzentrische Coextrusion erhalten, wobei der Strang anschließend geschnitten wird. Solche WC-Steine weisen einen Mantel und einen innenliegenden Kern auf. Ein weiterer Formungsschritt ist nicht vorgesehen. Auf Grund der Geometrie der beiden Zusammensetzungen löst sich der Mantel zuerst auf. Hierdurch kommt es zu Beginn der Verwendung zu einer verhältnismäßig starken Abgabe des ersten Aktivstoffes und zu einem späteren Zeitpunkt zu einer stärkeren Abgabe des zweiten Aktivstoffes. In der Regel ist allerdings eine gleichbleibende Abgabe der beiden Aktivstoffe über im Wesentlichen den gesamten Verwendungszeitraum gewünscht. Hierfür sind solche coextrudierten Rimblocks ungeeignet.

DE 10 2015 215 135 A1 beschreibt WC-Steine, die aus mindestens einer Masse und einem Kern bestehen.

Es war daher wünschenswert, einen formschönen und ästhetischen WC-Stein bereitzustellen, der unterschiedliche Aktivstoffe enthalten kann und der während seiner gesamten Lebensdauer gleichmäßig abgespült wird und möglichst wenig aufquillt.

Die Aufgabe wird durch einen erfindungsgemäßen WC-Stein gelöst aufweisend wenigstens eine erste Zusammensetzung und eine zweite Zusammensetzung, wobei sich die erste Zusammensetzung von der zweiten Zusammensetzung unterscheidet und die Herstellung des WC-Steins einen nicht-konzentrischen Coextrusionsschritt umfasst, wobei
die erste Zusammensetzung in räumlichem Kontakt mit einem Teilbereich der Seitenwandung des Durchganges steht und die zweite Zusammensetzung in räumlichem Kontakt mit einem anderen Teilbereich der Seitenwandung steht. Durch einen Formungsschritt das Extrudat unter Verwendung einer Abrollmaschine zu einer Kugel (502) geformt wird und der WC-Stein damit im Wesentlichen eine Kugelgeometrie aufweist, wobei die beiden Zusammensetzung auf der Oberfläche schlierenartig miteinander verwoben.

Im Zusammenhang mit der vorliegenden Erfindung steht der Begriff Extrusion für ein Verfahren, bei dem eine oder mehrere Zusammensetzungen unter Druck bevorzugt kontinuierlich aus einer Öffnung herausgepresst werden. Die Öffnung ist einem Fachmann auch als Profildüse bekannt. Der dabei entstehende Körper mit dem Querschnitt der Öffnung wird Extrudat genannt.

Im Zusammenhang mit der vorliegenden Erfindung steht der Begriff Coextrusion für ein Extrusionsverfahren, wobei wenigstens eine erste Zusammensetzung und eine zweite Zusammensetzung in einem Extrusionskopf oder auch Extruderkopf genannt zusammengeführt werden.

Der Extruderkopf bildet einen Durchgang, wobei ein Ende des Durchgangs durch eine Profildüse gebildet ist, durch die die zusammengeführten Zusammensetzungen als Extrudat heraustreten können.

Während der Extrusion kommt es im Extrusionskopf im Wesentlichen zu keiner vollständigen homogenen Vermischungen der ersten Zusammensetzung und der zweiten Zusammensetzung. Anders ausgedrückt bleibt die erste Zusammensetzung und die zweite Zusammensetzung dahingehend erhalten, dass diese im Extrudat noch voneinander unterschieden werden können. Dies schließt nicht aus, dass es im Kontaktbereich der ersten Zusammensetzung und der zweiten Zusammensetzung teilweise zu einer leichten Vermischung oder Vermengung der Zusammensetzungen kommen kann.

Der Durchgang im Extruderkopf weist eine Seitenwandung auf. Die erste Zusammensetzung und die zweite Zusammensetzung werden derart im Extruderkopf zusammengeführt, dass die erste Zusammensetzung in räumlichem Kontakt mit einem Teilbereich der Seitenwandung des Extruderkopfes steht und die zweite Zusammensetzung in räumlichem Kontakt mit einem anderen Teilbereich der Seitenwandung des Extruderkopfes steht.

Eine solche Anordnung unterscheidet sich insbesondere von einer konzentrischen Coextrusion, bei welcher eine erste Zusammensetzung vollständig in räumlichem Kontakt mit der Seitenwandung des Extruderkopfes steht und eine zweite Zusammensetzung konzentrisch innerhalb der ersten Zusammensetzung extrudiert wird, d.h. die zweite Zusammensetzung in keinem räumlichen Kontakt mit der Seitenwandung des Extruderkopfes steht. Im Extrudat ist die zweite Zusammensetzung damit konzentrisch von der ersten Zusammensetzung umschlossen. Das erfindungsgemäße Extrudat und damit auch der erfindungsgemäße WC-Stein unterscheidet sich damit deutlich von einem solchen aus dem Stand der Technik bekannten konzentrischen Extrudat.

WC-Stein gemäß den vorhergehenden Absätzen sind bevorzugt, wobei die erste Zusammensetzung einen ersten Aktivstoff aufweist und die zweite Zusammensetzung einen zweiten Aktivstoff aufweist, wobei sich der erste Aktivstoff und der zweite Aktivstoff voneinander unterscheiden.

Bevorzugt ist der erste Aktivstoff ein erster Farbstoff und der zweite Aktivstoff ein zweiter Farbstoff.

WC-Stein gemäß den vorhergehenden Absätzen sind bevorzugt, wobei der erste Aktivstoff ein erster Duftstoff ist und der zweite Aktivstoff ein zweiter Duftstoff ist.

Bevorzugt umfasst die Erfindung einen WC-Stein sowie ein Verfahren zur Herstellung eines WC-Steins, wobei die Herstellung des WC-Steins nach dem Coextrusionsschritt einen Formungsschritt umfasst.

Gemäß einer bevorzugten Ausführungsform, bilden die Zusammensetzungen im Wesentlichen zu gleichen Anteilen die Oberfläche des WC-Blocks.

Bevorzugt weist der WC-Stein gemäß der vorliegenden Erfindung wenigstens eine C₂ Symmetrie auf, d.h. der Stein ist dahingehend symmetrisch, dass als Symmetrieelement wenigstens eine zweizählige Rotationsachse vorliegt. Bevorzugt umfasst die Erfindung ferner einen WC-Stein sowie ein Verfahren zur Herstellung eines WC-Steins, wobei der WC-Stein rotationssymmetrisch ist, wobei als Symmetrieelement wenigstens eine C_{∞} Achse (C unendlich Achse) vorliegt, wobei selbstverständlich auch höhersymmetrische Geometrien umfasst sind (beispielsweise mehrere C_{∞} Achsen). Eine bevorzugte Geometrie ist der Zylinder, wobei sich dieser aufgrund seiner rotationssymmetrischen Geometrie gleichmäßig abspülen lässt, d.h. das Wasser kann insbesondere über den Zylindermantel gleichmäßig ablaufen. Eine solche Symmetrie ist insbesondere bei einem WC-Stein bevorzugt, der zwei Zusammensetzungen umfasst, die im Wesentlichen zu gleichen Anteilen die Oberfläche des WC-Blocks bilden. So kommt es in Zusammenspiel mit der Symmetrie des WC-Steins zu einer gleichzeitigen Freisetzung beider Zusammensetzungen in das Spülwasser. Dies steht im Gegensatz zu einer konzentrischen Geometrie, bei der zunächst der Mantel und erst dann der Kern des Zylinders abgespült werden.

Eine Kugelform ist ganz besonders bevorzugt, da diese aufgrund ihrer symmetrischen Geometrie den WC-Stein im Wesentlichen gleichmäßig abspülen lässt, d.h. das Wasser kann an allen Seiten gleichmäßig ablaufen. Eine solche Symmetrie ist insbesondere bei einem WC-Stein bevorzugt, der zwei Zusammensetzungen umfasst, die im Wesentlichen zu gleichen Anteilen die Oberfläche des WC-Blocks bilden. So kommt es in Zusammenspiel mit der Symmetrie des WC-Steins zu einer gleichzeitigen Freisetzung beider Zusammensetzungen in das Spülwasser.

Bevorzugt umfasst die Erfindung einen WC-Stein sowie ein Verfahren zur Herstellung eines WC-Steins, wobei durch den Formungsschritt das Extrudat zu einer Kugel geformt wird und der WC-Stein damit im Wesentlichen eine Kugelgeometrie aufweist.

In der Regel ist eine gleichbleibende Abgabe der beiden Aktivstoffe über im Wesentlichen den gesamten Verwendungszeitraum gewünscht. Durch einen Kugelformungsschritt werden die beiden Zusammensetzung auf der Oberfläche miteinander dahingehend "verwoben", dass die beiden Zusammensetzungen schlierenartig miteinander verwoben werden und damit völlig gleichmäßig über die Oberfläche verteilt sind. Eine solche Verteilung der Zusammensetzungen auf der Oberfläche des Steins lässt sich insbesondere durch gleichzeitiges Schneiden und Kugelformen eines Zylindrischen (nicht-konzentrischen) Coextrudates erreichen.

Eine Kugelform ist ferner besonders bevorzugt, da diese aufgrund ihrer hochsymmetrischen Geometrie der WC-Steine gleichmäßig abgespült wird. Dabei wird Spülwasser entlang der Oberfläche der Kugel gleichmäßig entlang geleitet und es kommt gleichmäßig mit der gesamten Oberfläche des Steins in Kontakt.

Bevorzugt umfasst die Erfindung einen WC-Stein sowie ein Verfahren zur Herstellung eines WC-Steins, wobei das Extrudat in kleinere Einheiten geschnitten wird.

Bevorzugt umfasst die Erfindung einen WC-Stein sowie ein Verfahren zur Herstellung eines WC-Steins, wobei das Extrudat in etwa gleich große kleinere Einheiten geschnitten wird.

Bevorzugt umfasst die Erfindung einen WC-Stein sowie ein Verfahren zur Herstellung eines WC-Steins, wobei der Durchgang im Wesentlichen zylinderförmig ausgebildet ist. Der Umfang des Teilzylinders des Extrudats, der der ersten Zusammensetzung entspricht, kann anders sein als der Umfang des Teilzylinders, der der zweiten Zusammensetzung entspricht. Hierdurch lässt sich das Aussehen und das Freisetzungsprofil des WC-Steins maßgeschneidert ändern. Möglich ist beispielsweise, dass die erste Zusammensetzung einen Teilzylinder bildet mit 3/4 Kreisumfang und die zweite Zusammensetzung einen Teilzylinder bildet mit 1/4 Kreisumfang. Möglich ist beispielsweise ferner, dass die erste Zusammensetzung einen Teilzylinder bildet mit 2/3 Kreisumfang und die zweite Zusammensetzung einen Teilzylinder bildet mit 1/3 Kreisumfang. Bevorzugt ist allerdings, dass die beiden Zusammensetzungen jeweils einen Halbzylinder bilden. Anders ausgedrückt, sofern der Durchgang im Wesentlichen zylinderförmig ausgebildet ist, weist der Extrusionsstrang bevorzugt Halbzylinder mit gleicher Größe auf.

Neben einer zylindrischen Stranggeometrie sind auch weitere Geometrien möglich wie z.B. dreieckig, rechteckig, sternförmig, oder auch anderweitig figürliche Formen. Hierbei kann ein weiterer Formungsschritt entfallen. Vorteilhafterweise lässt sich durch solche Variationen ein Freisetzungsprofil für Aktivstoffe der ersten Zusammensetzung im Vergleich zu der zweiten Zusammensetzung maßschneidern.

Bevorzugt umfasst die Erfindung einen WC-Stein sowie ein Verfahren zur Herstellung eines WC-Steins, wobei der Teilbereich der Seitenwandung des Durchgangs, mit dem die erste Zusammensetzung in Kontakt steht und der Teilbereich der Seitenwandung des Durchganges, mit dem die zweite Zusammensetzung in Kontakt steht, eine im Wesentlichen gleich große Fläche aufweisen.

Teilt man einen Zylinder durch eine Ebene, die seine Achse enthält, so entstehen zwei kongruente Halbzylinder. Wie beim Zylinder bestimmen der Radius und die Höhe den Halbzylinder. Sofern der Durchgang im Wesentlichen zylinderförmig ausgebildet ist, weist der Extrusionsstrang Halbzylinder mit gleicher Größe auf.

Gemäß einem weiteren Aspekt betrifft die Erfindung eine Vorrichtung aufweisend wenigstens einen WC-Stein gemäß einem der Ansprüche, wobei der wenigstens eine WC-Stein in einer Kammer der Vorrichtung vorliegt, wobei die Vorrichtung am Rand einer Toilette angebracht werden kann.

Vorzugsweise weist die Vorrichtung wenigstens zwei, bevorzugt wenigstens drei und weiter bevorzugt wenigstens vier WC-Steine auf.

Im Folgenden wird bespielhaft eine bevorzugte Vorrichtung beschreiben. Die Abgabevorrichtung besteht aus einem Behälter, welcher an seinem kopfseitigen Ende eine Einlassöffnung aufweist, durch die Spülwasser über das Spülwasserverteilelement in den Behälter eintreten kann. Das in den Behälter eingetretene Spülwasser löst etwas vom im Behälter bevorrateten WC-Reinigungsblock, wobei das nunmehr mit der entsprechenden Zubereitung beladene Spülwasser den Behälter über die Auslassöffnung verlässt und so ins Innere des Toilettenbeckens abgegeben wird.

Das Spülwasserverteilelement ist in der hier beschriebenen Ausführungsform plattenartig ausgeformt. Daneben sind aber beispielsweise auch wannenartige, rutschenartige oder rampenartige Ausformungen sowie beliebige Kombinationen derselben möglich. Das Spülwasserverteilelement greift in den Spülwasserstrom des Toilettenbeckens ein, wobei üblicherweise die Hauptströmungsrichtung des Spülwasserstroms in Schwerkraftrichtung nach unten gerichtet ist. Der Spülwasserstrom wird durch das Spülwasserverteilelement, welches ähnlich wie eine Prallplatte wirkt, gebrochen und über die Oberfläche des Spülwasserverteilelements verteilt. Die dem Spülwasserfluss zugewandte Oberfläche des Spülwasserverteilelements kann flüssigkeitsleitende und/oder -verteilende Strukturen aufweisen, wie beispielsweise quer und/oder längs verlaufende Rillen, Kapillaren oder Gitter.

Das Spülwasserverteilelement kann ferner eine Öffnung aufweisen, durch welche Spülwasser in die Einlassöffnung des Behälters einfließen kann.

Das gezeigte WC-Körbchen weist ein Halteelement auf, durch den das WC-Körbchen am Rand eines Toilettenbeckens durch einen Benutzer lösbar angebracht werden kann.

Der Halter weist ein erstes Federelement und ein zweites Federelement auf, wobei im in der Toilette eingebauten Zustand des WC-Spülers das erste Federelement einen im Wesentlichen vertikalen Federweg und das zweite Federelement einen im Wesentlichen horizontalen Federweg aufweist, wodurch eine verbesserte und flexiblere Fixierung des WC-Körbchens an Toiletten mit unterschiedlichen Beckenrandstärken und - ausgestaltungen ermöglicht ist.

Gemäß einer weiteren Ausführungsform weist die Abgabevorrichtung ein plattenartiges Spülwasserverteilelement auf, welches sich im eingebauten Zustand der Abgabevorrichtung in einem Toilettenbecken unmittelbar von unterhalb der Einlassöffnung des Behälters in Richtung des Toilettenrands erstreckt.

Gemäß einer weiteren Ausführungsform weist der Durchgang innen eine Querschnittsfläche auf, die bevorzugt etwa 1 bis 10 cm² beträgt und weiter bevorzugt 2 bis 5 cm² beträgt.

Gemäß einer weiteren Ausführungsform weist der Durchgang innen einen Durchmesser auf, der bevorzugt zwischen 15 mm und 30 mm, weiter bevorzugt zwischen 15 und 25 mm und weiter bevorzugt zwischen 18 mm und 22 mm beträgt.

Sofern der WC-Stein zu einer Kugel geformt wird, beträgt das Kugelvolumen bevorzugt 2 cm³ bis 20 cm³, weiter bevorzugt 5 cm³ bis 15 cm³ und weiter bevorzugt 8 cm³ bis 9 cm³.

Gemäß einer weiteren Ausführungsform beträgt der Kugeldurchmesser bevorzugt 10 mm bis 40 mm, weiter bevorzugt 15 mm bis 30 mm und weiter bevorzugt 22 mm bis 28 mm.

Gemäß einer weiteren Ausführungsform beträgt das Kugelgewicht bevorzugt 5 bis 30 g, weiter bevorzugt 5 bis 20 g und weiter bevorzugt 10 bis 15 g.

Gemäß einem weiteren Aspekt betrifft die Erfindung ein Verfahren zur Herstellung eines festen WC-Steins umfassend einen Coextrusionsschritt, wobei die wenigstens eine erste Zusammensetzung und eine zweite Zusammensetzung, vor dem Verlassen durch eine Profildüse, in einem Durchgang eines Extrusionskopfes derart zusammengeführt werden, dass die erste Zusammensetzung in räumlichem Kontakt mit einem Teilbereich der Seitenwandung des Durchganges steht und die zweite Zusammensetzung in räumlichem Kontakt mit einem anderen Teilbereich der Seitenwandung steht.

Gemäß einer bevorzugten Ausführungsform ist das Verfahren dahingehend ausgestaltetet, dass das Verfahren ferner umfasst: wenigstens einen Schneideschritt, wobei durch den Formungsschritt das Extrudat zu kleineren Einheiten geschnitten wird.

Gemäß einer weiteren bevorzugten Ausführungsform ist das Verfahren dahingehend ausgestaltetet, dass das Verfahren ferner umfasst: wenigstens einen Schneideschritt, wobei durch den Formungsschritt das Extrudat zu kleineren Einheiten geschnitten wird. Der Schneideschritt findet bevorzugt im Wesentlichen gleichzeitig mit einem Formungsschritt statt, wobei der Formungsschritt bevorzugt ein Kugelformungsschritt ist. Gemäß einer bevorzugten Ausführungsform ist das Verfahren dahingehend ausgestaltetet, dass das Verfahren ferner umfasst: einen Formungsschritt, wobei durch den Formungsschritt das Extrudat zu einer Kugel geformt wird und der WC-Stein damit im Wesentlichen eine Kugelgeometrie aufweist.

Bevorzugt erfolgt das Zuschneiden des Strangs in Abmessungen, die in allen drei Raumrichtungen ähnlich groß sind. Eine anschließende Ballformung bzw. Kugelformung ist möglich, aber nicht zwingen erforderlich.

Neben einer zylindrischen Stranggeometrie sind auch weitere Geometrien möglich wie z.B. dreieckig, rechteckig, sternförmig, oder auch anderweitig figürliche Formen. Hierbei ist vorteilhaft, dass ein weiterer Formungsschritt entfällt. Ferner lässt sich hierdurch auch eine Variation des Freisetzungsprofils erreichen.

Gemäß einer bevorzugten Ausführungsform ist das Verfahren dahingehend ausgestaltetet, dass der Schneidschritt und der Formungsschritt gleichzeitig durchgeführt werden. Dabei wird beispielsweise durch rotierende Walzen der Strang geschnitten und gleichzeitig durch die Walzenform zu einer Kugel geformt. Solche Verfahren sind für Extrudate mit einer einzigen Zusammensetzung aus der Herstellung für Schokoladenkugeln bekannt. Im Falle der vorliegenden Erfindung wurde überraschend festgestellt, dass solche Verfahren bei Extrudaten mit zwei Zusammenseztungen, die beiden Zusammensetzungen schlierenartig miteinander verweben.

Die folgenden Beispiele zeigen exemplarisch mögliche Zusammensetzungen V1, V2, V3, V4 und V5. Alle Mengenangaben sind dabei in Gew.-%:

| | V1 | V2 | V3 | V4 | V5 |
|---|---|---|---|---|---|
| C₁₀₋₁₃-lin. Alkylbenzolsulfonat-Na | 26 | -- | 12,4 | 21 | -- |
| Fettalkoholsulfat-Na | -- | 7,4 | -- | -- | -- |
| C₁₂- Fettalkoholsulfat-Na | -- | 17,4 | 12,4 | -- | -- |
| C₁₄₋₁₆-Olefinsulfonat-Na | 18 | -- | -- | 23 | 20 |
| C₁₆₋₁₈-Fettalkoholethoxylat 25 EO | 8 | 17 | 17 | -- | 17 |
| Cellulose | -- | 3 | -- | -- | -- |
| Trinatriumcitrat-Dihydrat | -- | 2 | 2 | 0,3 | 2 |
| Natriumsulfat | ad 100 | ad 100 | ad 100 | ad 100 | ad 100 |
| Natriumcarbonat | -- | 0,95 | 0,95 | -- | 0,95 |
| C₁₂₋₁₈-Fettsäuremonoethanolamid | -- | 8 | 15 | -- | 15 |
| Natriumsilikat | -- | -- | -- | 3 | -- |

Mittlerweise sind kugelförmige WC-Steine mit unterschiedlichsten Kombinationen von verschiedenen Tensiden bekannt. So können die Zusammensetzungen nur anionische Tenside, nur nicht-ionische Tenside oder Mischungen hiervon beinhalten. Auch kationische Tenside sind denkbar. Die angeführten Zusammensetzungen sind daher nur exemplarisch und nicht einschränkend zu verstehen.

Aus V1, V2, V3, V4 und V5 wird eine erste Zusammensetzung und eine zweite Zusammensetzung ausgewählt. Ferner werden der ersten Zusammensetzung und der zweiten Zusammensetzung Aktivstoffe hinzugefügt. F1 ist ein gelber Farbstoff und F2 ein blauer Farbstoff. P1 ist ein Zitronen Duftstoff und P2 ein Pinienduftstoff. Durch Variation der Zusammensetzungen und der Aktivstoffe ergeben sich die folgenden Kombinationen K1 bis K5:

| | Zusammensetzung 1 | Aktivstoffe | Zusammensetzung 2 | Aktivstoffe |
|---|---|---|---|---|
| Kombination K1 | V1 | F1 | V1 | F1 |
| Kombination K2 | V1 | F1, P1 | V4 | F2, P2 |
| Kombination K3 | V2 | F1, P1 | V3 | F2 |
| Kombination K4 | V2 | F2, P2 | V4 | F1, P2 |
| Kombination K5 | V2 | F2, P1 | V5 | F1, P1 |
| Kombination K6 | V3 | P1 | V4 | P2 |

Hieraus wird ein fester WC-Stein geformt, wobei die Herstellung des WC-Steins erfindungsgemäß einen Coextrusionsschritt umfasst, wobei die erste Zusammensetzung in räumlichem Kontakt mit einem Teilbereich der Seitenwandung eines Durchganges des Extruders steht und die zweite Zusammensetzung in räumlichem Kontakt mit einem anderen Teilbereich der Seitenwandung steht.

Der Durchgang des Extruders ist im Wesentlichen zylinderförmig ausgebildet. Die Zusammensetzungen werden zu im Wesentlichen gleichen Anteilen eingesetzt, d.h. das zylinderförmige Extrudat weist zwei in etwa gleich große Teilvolumina auf. Die Zusammensetzungen bilden also jeweils einen Halbzylinder, d.h. der zylindrische Extrusionsstrang ist durch zwei Halbzylinder mit gleicher Größe gebildet.

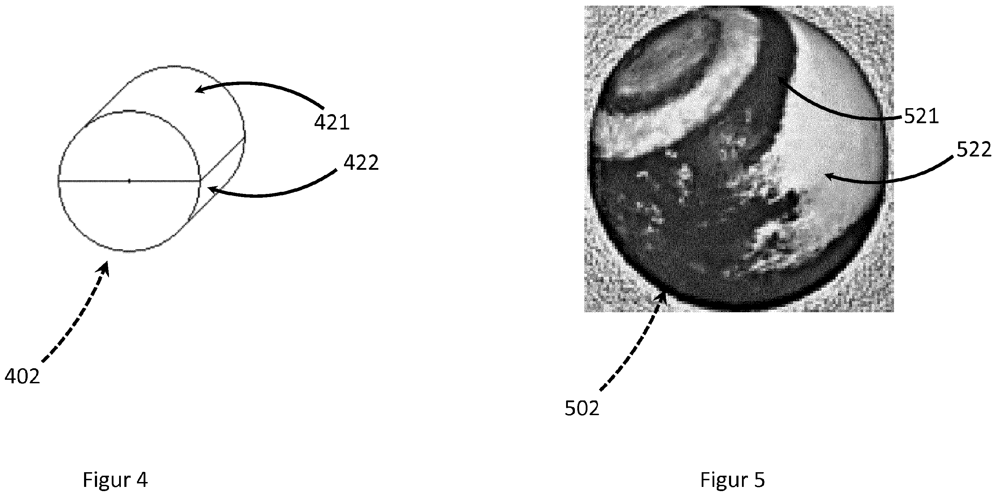

Die Steine wurden nach Extrusion einem Schneideschritt und anschließend einem Formungsschritt unterworfen wurden, wobei durch den Formungsschritt das Extrudat zu einer Kugel geformt wurde und der WC-Stein damit im Wesentlichen eine Kugelgeometrie aufwies. Mittels der erhaltenden WC-Steine wurde eine Vorrichtung erhalten, aufweisend vier Kammern, wobei jede Kammer mit einem der runden WC-Steine gefüllt wurde. Die Steine wiesen eine gut sichtbare und gleichmäßig strukturierte Marmorierung auf, d.h. die beiden Zusammensetzungen waren schlierenartig miteinander verwoben und nahmen etwa gleich große Flächen auf der Oberfläche des WC-Steins ein. Ein solcher WC-Stein ist in Figur 5 gezeigt. Anders als bei bekannten kugelförmigen Steinen, die durch eine konzentrische Koextrusion erhalten werden können, und damit eine Kern-Mantel Geometrie besitzen, wurde bei den hier erhaltenen Steinen eine gleichmäßige Freisetzung beider Zusammensetzungen in das Wasser, welches an der runden Kugeloberfläche gleichmäßig hinabfließen kann, ermöglicht.

Die Vorrichtung wird am Rand einer Toilette angebracht und wird mittels Spülen getestet, wobei bei allen WC Steinen ein im Wesentlichen gleichmäßiges Abspülen der beiden Zusammensetzungen und damit der verschiedenen in Aktivstoffe beobachtet werden kann.

Anhand der beigefügten Zeichnungen werden die geeigneten erfindungsgemäßen WC-Reinigungsblöcke erläutert. Im Einzelnen sind dargestellt:
- Fig. 1: Konzentrische Coextrusion aus dem Stand der Technik
- Fig. 2: Nicht-konzentrische Coextrusion gemäß der Erfindung
- Fig. 3: Vorrichtung für eine Nichtkonzentrische Coextrusion gemäß der Erfindung
- Fig. 4: WC-Stein gemäß der Erfindung
- Fig. 5: Weiterer WC-Stein gemäß der Erfindung

### Bezugszeichenliste:

105 Extruderkopf
110 Durchgang des Extruderkopfes
110a Seitenwandung des Extruderkopfes
121 erste Zusammensetzung
122 zweite Zusammensetzung
205 Extruderkopf
210 Durchgang des Extruderkopfes
210a Teil der Seitenwandung des Extruderkopfes
220b Teil der Seitenwandung des Extruderkopfes
221 erste Zusammensetzung
222 zweite Zusammensetzung
300 Vorrichtung zur Herstellung des erfindungsgemäßen WC-Blocks
305 Durchgang des Extruderkopfes
310 Durchgang des Extruderkopfes
310a Teilbereich der Seitenwandung des Extruderkopfes
310b Teilbereich der Seitenwandung des Extruderkopfes
312 Profildüse
320b Seitenwandung des Extruderkopfes
321 erste Zusammensetzung
322 zweite Zusammensetzung
330 Schnittstelle
402 WC-Block erhalten ohne Kugelformungsschritt
521 erste Zusammensetzung
522 zweite Zusammensetzung
502 WC-Block erhalten mit Kugelformungsschritt
521 erste Zusammensetzung
522 zweite Zusammensetzung

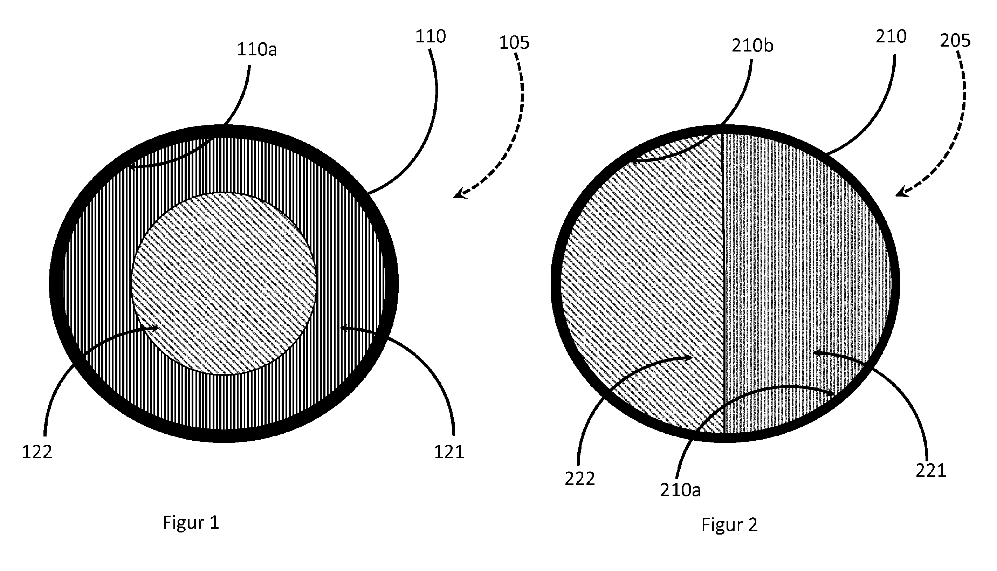

Figur 1 zeigt einen Extruderkopf (105) aufweisend einen Durchgang (110), der in einem Verfahren eingesetzt wird, um einen festen WC-Stein gemäß dem Stand der Technik herzustellen. Der Extruderkopf stellt ein Extrudat bereit aufweisend wenigstens eine erste Zusammensetzung (121) und eine zweite Zusammensetzung (122). Die erste Zusammensetzung und die zweite Zusammensetzung unterscheiden sich voneinander. Die Herstellung des WC-Steins umfasst einen Coextrusionsschritt der konzentrisch erfolgt. Bei der konzentrischen Extrusion steht die erste Zusammensetzung in räumlichem Kontakt mit der gesamten Seitenwandung des Durchganges (110a), während die zweite Zusammensetzung nicht in räumlichem Kontakt mit Seitenwandung des Durchganges steht.

Figur 2 zeigt einen Extruderkopf (205) aufweisend einen zylinderförmigen Durchgang (210), der in einem Verfahren eingesetzt wird um einen festen WC-Stein gemäß der Erfindung herzustellen. Der Extruderkopf stellt ein Extrudat bereit aufweisend wenigstens eine erste Zusammensetzung (221) und eine zweite Zusammensetzung (222). Die erste Zusammensetzung und die zweiten Zusammensetzung unterscheiden sich voneinander. Die Herstellung des WC-Steins umfasst einen Coextrusionsschritt, der nicht-konzentrisch erfolgt. Bei dieser erfindungsgemäßen Extrusion steht die erste Zusammensetzung in räumlichem Kontakt mit einem Teilbereich der Seitenwandung des Durchganges (210a), während die zweite Zusammensetzung mit einem anderen Teilbereich der Seitenwandung (210b) des zylinderförmigen Durchganges in Kontakt steht.

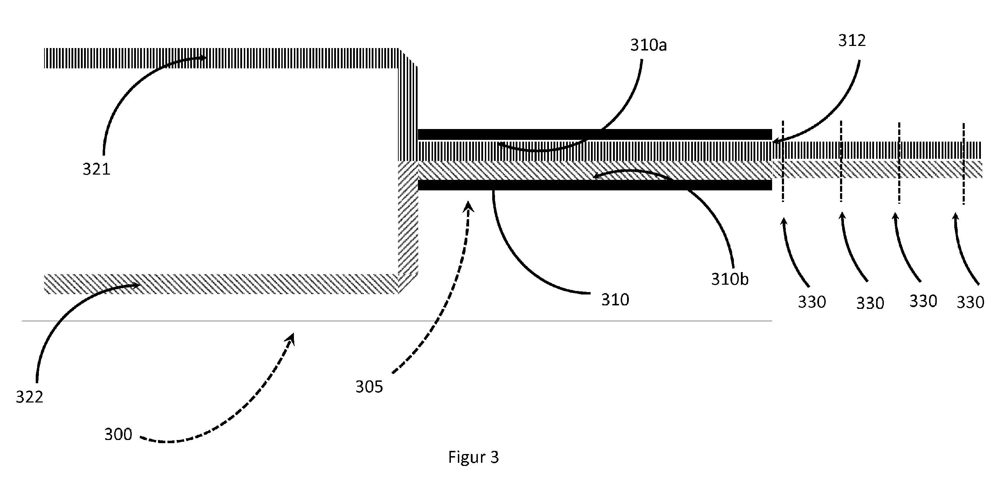

Figur 3 zeigt eine Vorrichtung (300) umfassend einen Extruderkopf (305) aufweisend einen Durchgang (310), die in einem Verfahren eingesetzt wird, um einen festen WC-Stein gemäß der Erfindung herzustellen. Der Extruderkopf stellt ein Extrudat bereit aufweisend wenigstens eine erste Zusammensetzung (321) und eine zweite Zusammensetzung (322). Die erste Zusammensetzung und die zweiten Zusammensetzung unterscheiden sich voneinander. Die Herstellung des WC-Steins umfasst einen Coextrusionsschritt, der nicht-konzentrisch erfolgt. Bei dieser erfindungsgemäßen Extrusion steht die erste Zusammensetzung in räumlichem Kontakt mit einem Teilbereich der Seitenwandung des Durchganges (310a), während die zweite Zusammensetzung mit einem anderen Teilbereich der Seitenwandung (310b) des Durchganges in Kontakt steht. Dabei werden die zunächst getrennten Stränge im Extruderkopf vereinigt. Der coextrudierte Strang wird durch eine Profildüse (312) aus dem Extruderkopf herausgepresst. Es folgen ein oder mehrere Schneideschritte. Gemäß der gezeigten Ausführungsform ist das Verfahren dahingehend ausgestaltetet, das durch mehrere Schnitte der Block in kleinere Einheiten getrennt wird. Die Schnitte können gleichzeitig oder zeitlich getrennt erfolgen.

Figur 4 zeigt einen erfindungsgemäßen WC-Stein (402), der mittels eines Coextrusionsschrittes, der nicht-konzentrisch erfolgt, hergestellt wurde. In der gezeigten speziellen Ausführungsform besteht der WC-Stein aus zwei Halbzylindern, wobei ein Halbzylinder aus einer ersten Zusammensetzung (421) besteht und der andere Halbzylinder aus einer zweiten Zusammensetzung (422) besteht. Der in Figur 4 gezeigte WC-Stein wurde ohne anschließenden Kugelformungsschritt erhalten, d.h. das zylindrische Extrudat wurde lediglich geschnitten und damit in kleinere Stücke zerteilt.

Figur 5 zeigt einen erfindungsgemäßen WC-Stein (502), der mittels eines Coextrusionsschrittes, der nicht-konzentrisch erfolgt, hergestellt wurde. In der gezeigten Ausführungsform wurde das Extrudat, welches aus zwei Halbzylindern besteht, wobei ein Halbzylinder aus einer ersten Zusammensetzung besteht (521) und der andere Halbzylinder aus einer zweiten Zusammensetzung besteht (522) in kleinere Stücke geschnitten und mittels eines Kugelformungsschritts geformt, d.h. das zylindrische Extrudat wurde geschnitten und zu einer Kugel verformt. Durch einen Kugelformungsschritt werden die beiden Zusammensetzung auf der Oberfläche miteinander dahingehend "verwoben", dass die beiden Zusammensetzungen schlierenartig miteinander vermengt sind und daher in etwa gleich große Flächen der Oberfläche des WC-Steins einnehmen. Eine solche Verteilung der Zusammensetzungen auf der Oberfläche des Steins lässt sich insbesondere durch gleichzeitiges Schneiden und Kugelformen eines zylindrischen (nicht-konzentrischen) Coextrudates erreichen. Dabei wird das zylindrische Coextrudat zwischen zwei rotierenden Rollen positioniert, die den länglichen Strang in die einzelnen WC-Steine "schneiden" und gleichzeitig jeden Stein zu einer Kugel formen. Solche Verfahren sind bekannt aus dem Bereich der Herstellung von Schokoladenkugeln. Hersteller entsprechender als Abrollmaschinen für Schokoladenkugeln bezeichneter Maschinen ist beispielsweise die Firma Krüger & Salecker Maschinenbau GmbH & Co. KG, wobei insbesondere der Typ ARM 0252 - 0601 bevorzugt ist.

## Patentansprüche

1. Ein fester kugelförmiger WC-Stein (402, 502) aufweisend wenigstens eine erste Zusammensetzung (221, 321, 421, 521) und eine zweite Zusammensetzung (222, 322, 422, 522), wobei sich die erste Zusammensetzung von der zweiten Zusammensetzung unterscheidet und die Herstellung des WC-Steins einen nicht-konzentrischen Coextrusionsschritt umfasst, wobei die erste Zusammensetzung in räumlichem Kontakt mit einem Teilbereich der Seitenwandung des Durchganges (210a, 310a) steht und die zweite Zusammensetzung in räumlichem Kontakt mit einem anderen Teilbereich der Seitenwandung (210b, 310b) steht, wobei durch einen Formungsschritt das Extrudat zu einer Kugel (502) unter Verwendung einer Abrollmaschine geformt wird und der WC-Stein damit im Wesentlichen eine Kugelgeometrie aufweist, wobei
die beiden Zusammensetzung auf der Oberfläche des kugelförmigen WC-steins schlierenartig miteinander verwoben sind.

2. WC-Stein gemäß dem vorhergehenden Anspruch, wobei die erste Zusammensetzung (221, 321, 421, 521) wenigstens einen ersten Aktivstoff aufweist und die zweite Zusammensetzung (222, 322, 422, 522) wenigstens einen zweiten Aktivstoff aufweist, wobei sich der erste Aktivstoff und der zweite Aktivstoff voneinander unterscheiden.

3. WC-Stein gemäß dem vorhergehenden Anspruch, wobei der erste Aktivstoff ein erster Farbstoff ist und der zweite Aktivstoff ein zweiter Farbstoff ist.

4. WC-Stein gemäß Anspruch 2 oder Anspruch 3, wobei der erste Aktivstoff ein erster Duftstoff ist und der zweite Aktivstoff ein zweiter Duftstoff ist.

## Claims

1. A solid spherical toilet block (402, 502) comprising at least a first composition ( 221, 321, 421, 521) and a second composition (222, 322, 422, 522), wherein the first composition differs from the second composition and the production of the toilet block comprises a non-concentric coextrusion step, wherein the first composition is in spatial contact with a sub-area of the side wall of the passage (210a, 310a) and the second composition is in spatial contact with another part of the side wall (210b, 310b), wherein, by means of a forming step, the extrudate is formed into a sphere (502) using a rolling machine and the toilet block thus has essentially a spherical geometry, wherein
the two compositions are interwoven with each other in a streak-like manner on the surface of the spherical toilet block.

2. Toilet block according to the preceding claim, wherein the first composition (221, 321, 421, 521) comprises at least one first active ingredient and the second composition (222, 322, 422, 522) comprises at least one second active ingredient, wherein the first active ingredient and the second active ingredient are different from each other.

3. Toilet block according to the preceding claim, wherein the first active ingredient is a first dye and the second active ingredient is a second dye.

4. Toilet block according to claim 2 or claim 3, wherein the first active ingredient is a first fragrance and the second active ingredient is a second fragrance.

## Revendications

1. Une pastille WC solide sphérique (402, 502) comprenant au moins une première composition ( 221, 321, 421, 521) et une deuxième composition (222, 322, 422, 522), la première composition étant différente de la deuxième composition et la fabrication de la pastille pour WC comprenant une étape de coextrusion non concentrique, la première composition étant en contact spatial avec une partie de la paroi latérale du passage (210a, 310a) et la deuxième composition étant en contact spatial avec une autre partie de la paroi latérale (210b, 310b), l'extrudat étant formé en une sphère (502) au moyen d'une machine à rouler, et la pastille WC présentant ainsi essentiellement une géométrie sphérique,
les deux compositions sont entrelacées sous forme de stries sur la surface de la pastille WC sphérique.

2. Bloc WC selon la revendication précédente, la première composition (221, 321, 421, 521) contenant au moins un premier agent actif et la deuxième composition (222, 322, 422, 522) contenant au moins un deuxième agent actif, le premier agent actif et le deuxième agent actif étant différents l'un de l'autre.

3. Brique WC selon la revendication précédente, le premier agent actif étant un premier colorant et le deuxième agent actif étant un deuxième colorant.

4. Brique WC selon la revendication 2 ou la revendication 3, le premier agent actif étant un premier parfum et le deuxième agent actif étant un deuxième parfum.
